(19) Europäisches Patentamt
European Patent Office
Office européen des brevets

(11) Veröffentlichungsnummer: **0 319 865 B1**

(12) **EUROPÄISCHE PATENTSCHRIFT**

(45) Veröffentlichungstag der Patentschrift: **22.06.94**

(51) Int. Cl.$^5$: **C08B 11/193**

(21) Anmeldenummer: **88120198.2**

(22) Anmeldetag: **03.12.88**

Die Akte enthält technische Angaben, die nach dem Eingang der Anmeldung eingereicht wurden und die nicht in dieser Patentschrift enthalten sind.

# (54) Carboxymethylsulfoethylcellulose und Verfahren zu ihrer Herstellung.

(30) Priorität: **11.12.87 DE 3742106**

(43) Veröffentlichungstag der Anmeldung:
**14.06.89 Patentblatt 89/24**

(45) Bekanntmachung des Hinweises auf die Patenterteilung:
**22.06.94 Patentblatt 94/25**

(84) Benannte Vertragsstaaten:
**DE FR IT NL SE**

(56) Entgegenhaltungen:
**GB-A- 659 506**
**GB-A- 1 072 021**
**JP-A-63 182 301**
**US-A- 2 811 519**

**CHEMICAL ABSTRACTS, Band 78, Nr. 24, 18. Juni 1973, Seite 67, Zusammenfassung-Nr. 149143s, Columbus, Ohio, US**

(73) Patentinhaber: **Wolff Walsrode Aktiengesellschaft**
**Postfach 15 15**
**D-29655 Walsrode(DE)**

(72) Erfinder: **Herzog, Dieter, Dr.**
**Hollige 19**
**D-3030 Walsrode(DE)**
Erfinder: **Balser, Klaus, Dr.**
**Geibelstrasse 24**
**D-3030 Walsrode(DE)**
Erfinder: **Szablikowski, Klaus, Dr.**
**Claudiusstrasse 5**
**D-3030 Walsrode(DE)**

(74) Vertreter: **Zobel, Manfred, Dr. et al**
**BAYER AG**
**Konzernverwaltung RP**
**Patente Konzern**
**D-51368 Leverkusen (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die Erfindung betrifft Carboxymethylsulfoethylcellulose mit bestimmten Eigenschaften sowie ein Verfahren zu deren Herstellung.

Carboxymethylsulfoethylcellulose (im folgenden CMSEC) ist ein wasserlöslicher ionischer Celluloseether. Die CMSEC zeigt im Vergleich zum industriell wichtigsten Celluloseether, der Carboxymethylcellulose (CMC), eine weitaus geringere Empfindlichkeit bei Zusatz von mehrwertigen Ionen oder pH-Wert-Schwankungen.

Ein weiteres wichtiges Kriterium bei der Verwendung von Celluloseethern ist die möglichst hohe Lösungsqualität. Die Qualität der Lösung wird durch Gelteilchen und Fasern negativ beeinflußt. Diese Gelteilchen und Fasern entstehen durch nicht oder unvollständig veretherte Cellulosepartikel. Sie können quantitativ durch eine Transmissionsmessung der wäßrigen Lösung des Celluloseethers bestimmt werden.

Sulfoethylcellulose ist z.B. aus der US-A-28 11 519 bekannt, ihre Verwendung als Bohrspülmittel aus der US-A-45 19 923.

CMSEC ist z.B. aus US-A-28 11 519 bekannt. In SU 79 40 98 ist die Verwendung von CMSEC als Viskositätsbildner für Textildruckfarben beschrieben. Es wird dort die Verwendung von CMSEC mit einem $DS_{Carboxymethyl}$ von 0,4 bis 0,55 und einem $DS_{Sulfoethyl}$ von 0,1 bis 0,25 als Viskositätsbildner in Textildruckfarben beansprucht. Unter $DS_{Carboxymethyl}$ wird im folgenden die durchschnittliche Substitution einer Glukoseeinheit der Cellulose mit einer Carboxymethylgruppe verstanden. Unter $DS_{Sulfoethyl}$ wird im folgenden die durchschnittliche Substitution einer Glukoseeinheit der Cellulose mit Sulfoethylgruppen verstanden. Die Lösungsqualität dieser hier beschriebenen CMSEC ist jedoch schlecht. Für diese CMSEC wird eine Transmission von 89,5 % in 0,5 %iger wäßriger Lösung angegeben.

Aufgabe der Erfindung war es, eine CMSEC zu finden, die in einem weiten Viskositätsbereich und in einer breiten Variation des Substitutionsgrades Carboxymethyl und des Substitutionsgrades Sulfoethyl eine verbesserte Lösungsqualität besitzt.

Gegenstand der Erfindung ist eine Carboxymethylsulfoethylcellulose (CMSEC) mit

1. einem Substitutionsgrad $DS_{Sulfoethyl}$ von 0,1 bis 1 und einem Substitutionsgrad $DS_{Carboxymethyl}$ von 0,3 bis 1,2, wobei die Substitutionsgrade folgender Gleichung genügen:

$$0{,}5 \leqq DS_{Sulfoethyl} + DS_{Carboxymethyl} \leqq 1{,}6,$$

2. einer Viskosität von 2 gew.-%iger Lösung von 5 mPa•s bis 60.000 mPa•s (gemessen bei einem Schergefälle von 2,5 $s^{-1}$ und 20 °C) und
3. einer Transmission in 2 gew.-%iger wäßriger Lösung von T >95 % (Wellenlänge des eingesetzten Lichtes: $\lambda$ = 550 nm, optische Weglänge der Küvette = 10 mm), erhältlich durch Veretherung von Cellulose in alkalischer Lösung mit wenigstens einer eine Sulfoalkylgruppe übertragenden Verbindung SA und wenigstens einer eine Carboxymethylgruppe übertragenden Verbindungen CM, wobei
   a) Cellulose, in einem sekundären oder tertiären Alkohol suspendiert wird,
   b) die Suspension zusammengegeben wird mit der Verbindung SA und/oder CM, unter Erhalt von pH 0 bis 9,
   c) anschließend durch Zugabe von Alkali alkalisch gestellt wird auf pH 13 bis 14,
   d) die Veretherung bei einer Temperatur zwischen 55 bis 100 °C durchgeführt wird und
   e) gegebenenfalls anschließend die Verbindung CM und/oder SA zugegeben wird, bei Temperaturen von 15 bis 100 °C und pH 10 bis 14 unter Bildung einer CMSEC.

In einer besonders bevorzugten Ausführungsform beträgt der Substitutionsgrad $DS_{Sulfoethyl}$ 0,3 bis 1,0 und der Substitutionsgrad $DS_{Carboxymethyl}$ 0,3 bis 1,2.

Gegenstand der Erfindung ist weiterhin ein Verfahren zur Herstellung einer CMSEC durch Veretherung von Cellulose in alkalischer Lösung mit wenigstens einer eine Sulfoalkylgruppe übertragenden Verbindung SA und wenigstens einer eine Carboxymethylgruppe übertragenden Verbindungen CM, dadurch gekennzeichnet, daß

a) Cellulose, beispielsweise in Form von fein gemahlenem Zellstoff, suspendiert wird, in einem sekundären oder tertiären Alkohol,
b) die Suspension zusammengegeben wird mit der Verbindung SA und/oder CM, vorzugsweise unter Erhalt von pH 0 bis 9 ,
c) anschließend, durch Zugabe von Alkali, alkalisch gestellt wird, vorzugsweise auf pH 13 bis 14,
d) die Veretherung durchgeführt wird, bei einer Temperatur zwischen 55 bis 100 °C,
e) gegebenenfalls anschließend die Verbindung CM und/oder SA zugegeben wird, bei Temperaturen von 15 bis 100 °C und pH 10 bis 14 unter Bildung einer CMSEC.

In einer besonders bevorzugten Ausführungsform des Verfahrens wird im Reaktionsschritt b) nur SA und im Reaktionsschritt e) nur CM zugesetzt. In einer anderen bevorzugten Ausführungsform wird im Reaktionsschritt b) nur CM und im Reaktionsschritt e) nur SA als wirksame Substanz zugesetzt.

Bevorzugte Sulfoalkyl-übertragende Verbindungen SA sind Chlorethansulfonsäure, Bromethansulfonsäure, Vinylsulfonsäure und deren Salze, insbesondere Salze der Vinylsulfonsäure, insbesondere der Natriumsalze.

Besonders bevorzugte Carboxymethyl-übertragende Verbindungen CM sind Chloressigsäure, Bromessigsäure und deren Salze, insbesondere Chloressigsäure.

Eine besonders bevorzugte Ausführungsform geht von einer gereinigten Carboxymethylcellulose, wie sie nach bekanntem Stand der Technik hergestellt wird, als Ausgangssubstanz für eine durch Weiterveretherung erhaltene CMSEC aus. In diesem erfindungsgemäßen Verfahren erfolgt die Zugabe der wäßrigen Lösung des Alkylierungsmittels vinylsulfonsaures Natrium vor der Alkalisierung der Carboxymethylcellulose. Diese Alkalisierung der Carboxymethylcellulose kann schonender erfolgen als es bei dem Einsatz von Zellstoff der Fall ist, d.h. daß geringere Mengen an Alkalihydroxiden eingesetzt werden können, und auch die Alkalisierzeit abgekürzt werden kann. Eine gewisse Mindestmenge an Alkalihydroxiden ist jedoch in jedem Falle erforderlich, da katalytische Mengen für die Alkylierung mit dem vinylsulfonsauren Natrium notwendig sind.

Überraschenderweise führt die Zugabe einer wäßrigen Lösung des als Alkylierungsmittel fungierenden vinylsulfonsauren Natriums zur in einem sekundären oder tertiären Alkohol suspendierten Cellulose vor der Alkalisierung zu einem Produkt mit guten Lösungsqualitäten. Dabei werden gute Chemikalienausbeuten, bezogen auf das vinylsulfonsaure Natrium, erzielt. Gemahlenes Cellulosepulver (Chemiezellstoff) z.B. Baumwoll-Linters, gereinigter Fichtensulfitzellstoff, gereinigter Fichtensulfatzellstoff wird in einem sekundären oder tertiären Alkohol, vorzugsweise Isopropanol oder tert.-Butanol, suspendiert. Die gewünschte Endviskosität der Produkte wird durch Ausschluß oder Zugabe von oxidierenden Substanzen, wie z.B. Luft, $H_2O_2$, Metalloxide und die Auswahl der eingesetzten Zellstoffe eingestellt. Diese Einstellung der Endviskosität ist Stand der Technik und jedem Fachmann geläufig. Verwiesen sei in diesem Zusammenhang auf das Kapitel "Celluloseether" in Ullmann's Encyklopädie der technischen Chemie, Band 9, Seite 192-212 (Verlag Chemie, Weinheim, 1975).

In dem erfindungsgemäßen Verfahren wird zur Herstellung der erfindungsgemäßen Produkte die Zugabe einer wäßrigen Lösung des Alkylierungsmittels insbesondere von vinylsulfonsaurem Natrium vor der Alkalisierung zu dem suspendierten Zellstoff durchgeführt und im allgemeinen 5 bis 60 Minuten, vorzugsweise 10 bis 30 Minuten, gerührt, bevor die Alkalisierung erfolgt. Dadurch erfolgt eine besonders gleichmäßige Verteilung des Alkylierungsmittels auf der Cellulose.

Durch Zugabe von Alkalihydroxiden sowie evtl. einer weiteren Menge an Wasser wird die Cellulose in die aktivierte Alkalicellulose überführt. Nach einer 60 bis 180minütigen Alkalisierzeit bei 0 bis 35 °C erfolgt im allgemeinen die Alkylierung durch Erhöhung der Temperatur auf Werte zwischen 55 und 100 °C, vorzugsweise auf 60 bis 80 °C. Man führt im allgemeinen die Alkylierung innerhalb eines Zeitraumes von 30 bis 300 Minuten, vorzugsweise 45 bis 180 Minuten, durch. Dann wird z.B. die Chloressigsäure kristallin oder als 80 %ige wäßrige Lösung hinzugegeben, und es wird bei einer Temperatur zwischen 55 und 100 °C, vorzugsweise 55 und 80 °C, weiterverethert. Die Zugabe der Chloressigsäure kann bei der vorhandenen Reaktionstemperatur erfolgen, oder aber nach Abkühlung des Reaktionsgemisches auf Werte von 15 bis 35 °C.

Die Veretherung in der zweiten Stufe kann 30 bis 600 Minuten dauern, vorzugsweise 45 bis 210 Minuten. Wenn nötig, wird das Reaktionsgemisch durch Zugabe von Säuren (z.B. Ameisensäure, Essigsäure, Salzsäure) neutralisiert. Dieses ist immer dann der Fall, wenn die Menge an eingesetzter Chloressigsäure nicht so groß ist, daß die gesamten Alkalihydroxide durch Reaktion mit der Chloressigsäure neutralisiert werden.

Das so erhaltene Produkt wird im allgemeinen vom Slurry-Medium abgetrennt (durch Zentrifugation oder Filtration) und durch Waschen mit Alkoholen oder Alkohol-Wasser-Gemischen (vorzugsweise Methanol bzw. Methanol-Wasser-Gemischen) von anhaftenden Salzen gereinigt.

Die erhaltenen erfindungsgemäßen Produkte sind geeignet als Verdicker oder Wasserrückhalter in den Bereichen Erdöl, Baustoffe sowie als Verdicker oder Stabilisator von Waschmitteln und Kosmetika. Des weiteren ist die CMSEC geeignet als Dispergier- und Suspendiermittel sowie als Hilfsmittel und Viskositätsbildner in wäßrigen Systemen, wie z.B. als Stabilisator, Dispergier- und Suspendiermittel bei Emulsions- und Suspensionspolymerisationen. Desgleichen ist die Verwendung als Verdicker und Stabilitator in tensidhaltigen Systemen, wie z.B. Reinigungsmitteln und Kosmetika, möglich.

Die in den folgenden Beispielen und Tabellen angegebenen Teile sind Gewichtsteile.

Die Messung der Viskositäten erfolgte mit einem Rotationsviskosimeter Haake, Typ RV 100, System M 500, Meßeinrichtung MV, nach DIN 53 019, bei einer Schergeschwindigkeit D = 2,5 $sec^{-1}$, bei einer Temperatur von 20 °C. Es wurden jeweils 2 gew.-%ige Lösung in destilliertem Wasser gemessen.

Die Transmissionsmessungen erfolgten in einem Hitachi-Spektralphotometer, Modell 101, Hitachi Ltd., Tokio/Japan. Es wurde eine Glasküvette mit 10 mm optischer Weglänge verwendet. Die verwendete Wellenlänge betrug 550 nm.

Beispiel 1

127,4 Teile feingemahlener (0,02 bis 0,5 mm) gebleichter, veredelter Fichtensulfitzellstoff werden in einem thermostatisierbaren Reaktor mit geeignetem Rührer unter Luftatmosphäre in 2.178 Teilen Isopropanol suspendiert und mit 159,34 Teilen einer 51,3 % vinylsulfonsauren Natriumsalzlösung in Wasser versetzt und 15 Minuten gerührt. Dann werden 75,46 Teile Natriumhydroxid gelöst in 147,4 Teilen Wasser zugegeben, und es wird 80 Minuten bei 25 bis 30 °C alkalisiert. Dann erwärmt man innerhalb von 60 Minuten auf 70 °C. Die Reaktionstemperatur von 70 °C wird 120 Minuten lang gehalten. Anschließend gibt man 92,34 Teile einer 80 gew.-%igen Lösung Chloressigsäure in Wasser zu und hält die Temperatur für weitere 90 Minuten auf 70 °C. Danach wird das Produkt filtriert und fünfmal mit je 2.000 Teilen einer Mischung aus 3 Anteilen Wasser und 7 Anteilen Methanol und abschließend noch einmal mit 2.000 Teilen reinem Methanol gewaschen. Das Produkt wird an der Luft getrocknet.

Beispiele 2 bis 6

Die Herstellung erfolgt analog Beispiel 1, wobei die gesamte Reaktion unter Stickstoffatmosphäre durchgeführt wird. Die genauen Produktherstelldaten sind Tabelle 1 zu entnehmen.

Beispiel 7

127,33 Teile gereinigter Fichtensulfitzellstoff werden in einem thermostatisierbaren, geschlossenen Reaktionsgefäß mit geeignetem Rührwerk unter Stickstoffatmosphäre in 2.178 Teilen Isopropanol suspendiert. Dazu gibt man 75,46 Teile Natriumhydroxid, gelöst in 225 Teilen Wasser. Die so erhaltene Mischung wird bei 25 bis 30 °C gerührt. Dabei erhält man aus dem Zellstoff die aktivierte Alkalicellulose.

Danach werden 80,44 g einer 80 gew.-%igen Lösung Chloressigsäure in Wasser zugegeben, und es wird in 30 Minuten auf 70 °C erwärmt. Diese Temperatur wird 90 Minuten lang gehalten. Dann werden 50 g Eisessig zugegeben, um die überschüssige Natronlauge zu neutralisieren. Das Produkt wird abfiltriert und wiederholt mit 70 %igem Methanol gewaschen, bis kein Kochsalz in der Reinigungsflüssigkeit mehr nachweisbar ist. Das Produkt wird an der Luft getrocknet. Das erhaltene Produkt besitzt eine Restfeuchte von 16,22 % an Wasser und einen Substitutionsgrad $DS_{Carboxymethyl}$ von 0,47.

Von diesem Produkt werden 119,12 Teile in 2.237 Teilen Isopropanol in einem Reaktionsgefäß, wie dem oben beschriebenen, unter Stickstoffatmosphäre suspendiert. Dazu werden 42,9 Teile einer 30,3 gew.-%igen vinylsulfonsauren Natriumsalzlösung gegeben, und es wird 15 Minuten gerührt. Man gibt 20 Teile Natriumhydroxid, gelöst in 120 Teilen Wasser hinzu und alkalisiert 60 Minuten lang bei 25 bis 30 °C. Danach wird in 60 Minuten auf 70 °C erwärmt, und diese Temperatur wird 150 Minuten gehalten. Zur Neutralisation des Ansatzes werden 30 Teile Essigsäure zugegeben. Das Produkt wird abfiltriert und je fünfmal mit 2.000 Teilen einer Mischung aus 7 Anteilen Methanol und 3 Anteilen Wasser gewaschen. Anschließend wird das Produkt an der Luft getrocknet.

Beispiel 8

Die Reaktion erfolgt analog Beispiel 7, wobei die Menge an eingesetztem vinylsulfonsauren Natriumsalz 85,81 Teile einer 30,3 %igen Lösung beträgt. Nach der Zugabe von vinylsulfonsauren Natriumsalz-Lösung werden 20 Teile Natriumhydroxid in 90,2 Teilen Wasser gelöst und nach einer Zeit von 15 Minuten zugegeben.

Die Produktdaten der erfindungsgemäßen Produkte sind in Tabelle 2 zusammengefaßt.

Tabelle 1

Herstellbedingungen

Beispiel 1 bis 6

Erläuterungen Tabelle 2

| | |
|---|---|
| VSSNa: | vinylsulfonsaures Natrium |
| NaOH: | Natriumhydroxid |
| $H_2O$: | Wasser |
| 80 gew.-%ige CES-Lösung: | Chloressigsäure-Lösung, 80 gew.-%ig |
| Reaktionszeit I: | Reaktionszeit für die Alkylierung mit vinylsulfonsaurem Natrium |
| Reaktionszeit II: | Reaktionszeit für die Alkylierung mit Chloressigsäure, nach der Zugabe der 80 igen Chloressigsäurelösung |
| Linters: | Chemiezellstoff aus gemahlenem, gebleichtem Baumwoll-Linters, Teilchengröße <0,4 mm |
| Fichte: | Chemiezellstoff aus gemahlenem, gebleichtem Fichten-Zellstoff, Teilchengröße <0,4 mm |
| $N_2$: | die gesamte Reaktion wurde unter Stickstoffatomosphäre durchgeführt |
| Luft: | die gesamte Reaktion wurde unter Luftatmosphäre durchgeführt |

Tabelle 2

Produktdaten

<u>Beispiel 1 bis 8</u>

<u>Erläuterungen Tabelle 3</u>

| | |
|---|---|
| Trockengehalt: | Rohprodukt (lufttrocken) abzüglich Feuchte, Angabe in Prozent |
| DS: | Substitutionsgrad, Anzahl der Substituenten pro Anhydroglycose-Baustein |
| Chemikalienausbeute: | Menge des zum Produkt umgesetzten Alkylierungsmittels, Angabe in Prozent des eingesetzten vinylsulfonsauren Natriums |
| Transmission: | Anteil des durchstrahlenden Lichtes in Prozent des eintretenden Lichtes beim Durchgang durch eine mit einer 2 %igen Celluloseether-Lösung gefüllten Küvette. Optische Weglänge der Küvette = 10 mm, eingesetzte Wellenlänge $\lambda$ = 550 nm |

**Tabelle 1** Herstellbedingungen

| Beispiel Nr. | iPrOH [Teile] | MeOH [Teile] | Zellstoff [Teile] | Zellstoff Art | VSSNa-Lsg. [Teile] | Konz. der VSSNa-Lsg. [Gew-%] | NaOH [Teile] | $H_2O$ [Teile] | 80 gew.-%ige CES-Lsg. [Teile] | Aufheizzeit [min] | Reaktionszeit I [min] | Reaktionszeit II [min] | Reaktionstemperatur [°C] | Atmosphäre |
|---|---|---|---|---|---|---|---|---|---|---|---|---|---|---|
| 1 | 2.178 | - | 127,41 | Fichte | 79,67 | 51,3 | 75,46 | 186,2 | 110,81 | 60 | 120 | 90 | 70 | Luft |
| 2 | 2.178 | - | 127,41 | Fichte | 278,85 | 51,3 | 75,46 | 135,8 | 92,34 | 60 | 120 | 90 | 70 | Luft |
| 3 | 2.178 | - | 127,41 | Linters | 159,34 | 51,3 | 75,46 | 147,4 | 110,81 | 60 | 120 | 90 | 70 | Luft |
| 4 | 2.178 | - | 127,41 | Linters | 278,85 | 51,3 | 75,46 | 89,2 | 92,34 | 60 | 120 | 90 | 70 | N2 |
| 5 | 2.178 | - | 127,41 | Linters | 159,34 | 51,3 | 75,46 | 147,4 | 110,81 | 60 | 120 | 90 | 70 | N2 |
| 6 | 2.178 | - | 127,41 | Linters | 79,67 | 51,3 | 75,46 | 186,2 | 110,81 | 60 | 120 | 90 | 70 | N2 |

**Tabelle 2 Produktdaten**

| Beispiel Nr. | Trockengehalt [%] | Viskosität 2 %ig in dest. $H_2O$ [mPa·s] | DS Sulfoethyl | DS Carboxymethyl [%] | Transmission [%] | Chemikalienausbeute [%] |
|---|---|---|---|---|---|---|
| 1 | 83,3 | 59 | 0,53 | 0,74 | 97,8 | 66,25 |
| 2 | 80,3 | 127 | 0,8 | 0,48 | 97,7 | 57,14 |
| 3 | 84,11 | 39.058 | 0,24 | 0,72 | 95,9 | 60 |
| 4 | 85,29 | 40.850 | 0,1 | 0,8 | 95,1 | 50 |
| 5 | 85,62 | 38.342 | 0,32 | 0,59 | 96,5 | 53,33 |
| 6 | 85,36 | 31.677 | 0,34 | 0,74 | 96,6 | 56,66 |
| 7 | 83,9 | 309 | 0,12 | 0,47 | 97 | 60 |
| 8 | 81,1 | 178 | 0,21 | 0,47 | 96 | 52,5 |

Beispiel 9

Tabelle 3 zeigt das Viskositätsverhalten einer Reihe von erfindungsgemäßen Produkten im Vergleich zu einer handelsüblichen Carboxymethylcellulose in einem Mischsalzsystem. Dabei ist zu erkennen, daß die erfindungsgemäßen Produkte eine deutlich höhere Viskosität im Mischsalzsystem zeigen als die Carboxymethylcellulose. Um eine bessere Vergleichbarkeit zu erreichen, wurde die Viskosität im Mischsalzsystem auf die Viskosität in destilliertem Wasser bezogen. Sie wird in % der Viskosität, die in destilliertem Wasser

gemessen wurde, angegeben.

Tabelle 3

| Viskositätsverhalten in Mischsalzsystemen | | | |
|---|---|---|---|
| Beispiel | Viskosität dest. Wasser (mPa•s) | Faktor Mischsalzsystem Raumtemperatur (%) | Faktor Mischsalzsystem 80° C (%) |
| 1 | 104,5 | 103,8 | 98,1 |
| 2 | 99,0 | 104,0 | 97,0 |
| 3 | 92,0 | 102,2 | 96,2 |
| 4 | 94,5 | 105,8 | 98,4 |
| CMC | 104,0 | 95,2 | 89,9 |

Erläuterungen Tabelle 3:

Die Viskositäten wurden von 1 gew.-%igen Lösungen gemessen. Zur Messung verwendet wurde ein Rotationsviskosimeter vom Typ Baroid Variable Speed Rheometer Nr. 286. Die Mesung wurde gemäß der Norm des American Petroleum Istitute gemessen (API, RP 13 B, 6th Edition, April 1986, issued by American Petroleum Institute, Production Department, 300 Carreegan Tower Building, Dallas, Texas 75201, USA). Die in Spalte 3 aufgeführten Messungen erfolgten nach 16 Stunden Lagerung bei Raumtemperatur. Die in Spalte 4 aufgeführten Messungen erfolgten nach 24 Stunden Lagerung bei Raumtemperatur und anschließender 16stündiger Lagerung bei 80°C. Die Viskositätsmessung erfolgte bei 25°C und einem Schergefälle von 1.021 $sec^{-1}$.

Die in Spalte 3 und 4 angegebene relative Viskosität im Mischsalzsystem (Angabe in Prozent) bezieht sich auf die Viskosität des gleichen Produktes in destilliertem Wasser:

$$\text{Faktor Mischsalzsystem} = \frac{\text{Viskosität im Mischsalzsystem (mPa·s)}}{\text{Viskosität in dest. } H_2O \text{ (mPa·s)}} \cdot 100\ \%$$

Zusammensetzung des Mischsalzsystems:

$$\begin{aligned} & 60\ g\ NaCl \\ + \ & 20\ g\ CaCl \cdot 2\ H_2O \\ + \ & 20\ g\ MgCl_2 \cdot 6\ H_2O \\ + \ & 1.000\ g\ \text{Wasser} \end{aligned}$$

Bei der als Vergleich aufgeführten Carboxymethylcellulose handelt es sich um eine handelsübliche Carboxymethylcellulose mit einem Substitutionsgrad von DS = 0,9, wie sie für Tiefbohrungen eingesetzt wird.

**Patentansprüche**

**1.** Carboxymethylsulfoethylcellulose (CMSEC) mit

1. einem Substitutionsgrad $DS_{Sulfoethyl}$ von 0,1 bis 1 und einem Substitutionsgrad $DS_{Carboxymethyl}$ von 0,3 bis 1,2, wobei die Substitutionsgrade folgender Gleichung genügen:

$$0,5 \leqq DS_{Sulfoethyl} + DS_{Carboxymethyl} \leqq 1,6,$$

2. einer Viskosität von 2 gew.-%iger Lösung von 5 mPa.s bis 60.000 mPa.s (gemessen bei einem Schergefälle von 2,5 $s^{-1}$ und 20 °C) und
3. einer Transmission in 2 gew.-%iger wäßriger Lösung von T > 95 % (Wellenlänge des eingesetzten Lichtes: λ = 550 nm, optische Weglänge der Küvette = 10 mm), erhältlich

durch Veretherung von Cellulose in alkalischer Lösung mit wenigstens einer eine Sulfoalkylgruppe übertragenden Verbindung SA und wenigstens einer eine Carboxymethylgruppe übertragenden Verbindungen CM, wobei

a) Cellulose, in einem sekundären oder tertiären Alkohol suspendiert wird,
b) die Suspension zusammengegeben wird mit der Verbindung SA und/oder CM, unter Erhalt von pH 0 bis 9,
c) anschließend durch Zugabe von Alkali alkalisch gestellt wird auf pH 13 bis 14,
d) die Veretherung bei einer Temperatur zwischen 55 bis 100 °C durchgeführt wird und
e) gegebenenfalls anschließend die Verbindung CM und/oder SA zugegeben wird, bei Temperaturen von 15 bis 100 °C und pH 10 bis 14 unter Bildung einer CMSEC.

**2.** Carboxymethylsulfoethylcellulose gemäß Anspruch 1, erhältlich dadurch, daß im Reaktionsschritt b) nur die Verbindung SA und im Reaktionsschritt e) nur die Verbindung CM zugesetzt wird.

**3.** Carboxymethylcellulose gemäß Anspruch 1, erhältlich dadurch, daß im Reaktionsschritt b) nur CM und im Reaktionsschritt e) nur SA zugesetzt wird.

**4.** Verfahren zur Herstellung einer Carboxymethylsulfoethylcellulose CMSEC gemäß Anspruch 1 durch Veretherung von Carboxymethylcellulose CMC in alkalischer Lösung mit wenigstens einer eine Sulfoalkylgruppe übertragenen Verbindung SA, dadurch gekennzeichnet, daß

a) CMC in einem sekundären oder tertiären Alkohol suspendiert wird,
b) die Suspension zusammengegeben wird mit der Verbindung SA unter Erhalt von pH 0 bis 9,
c) anschließend, durch Zugabe von Alkali, alkalisch gestellt wird, auf pH 13 bis 14,
d) die Veretherung bei einer Temperatur zwischen 55 bis 100 °C unter Bildung einer CMSEC durchgeführt wird.

## Claims

**1.** Carboxymethyl sulfoethyl cellulose (CMSEC) having

1. a degree of substitution $DS_{sulfoethyl}$ of 0.1 to 1 and a degree of substitution $DS_{carboxymethyl}$ of 0.3 to 1.2, the degrees of substitution satisfying the following equation:

$$0.5 \leqq DS_{sulfoethyl} + DS_{carboxymethyl} \leqq 1.6,$$

2. a viscosity in the form of a 2% by weight solution of 5 mPa.s to 60,000 mPa.s (as measured at a shear rate of 2.5 $s^{-1}$/20 °C) and
3. a transmission T in the form of a 2% by weight aqueous solution of 95% (wavelength of the light used: λ = 550 nm, optical wavelength of the cell = 10 mm),

obtainable by etherification of cellulose in alkaline solution with at least one compound SA transferring a sulfoalkyl group and at least one compound CM transferring a carboxymethyl group,

a) cellulose being suspended in a secondary or tertiary alcohol,
b) the suspension being combined with the compound SA and/or CM at pH 0 to 9 and then
c) alkalized to pH 13-14 by addition of alkali,
d) the etherification being carried out at a temperature of 55 to 100 °C and
e) the compound CM and/or SA optionally being added at temperatures of 15 to 100 °C/pH 10 to 14 to form a CMSEC.

**2.** Carboxymethyl sulfoethyl cellulose as claimed in claim 1 obtainable by adding only the compound SA in reaction step b) and only the compound CM in reaction step e).

3. Carboxymethyl sulfoethyl cellulose as claimed in claim 1 obtainable by adding only CM in reaction step b) and only SA in reaction step e).

4. A process for the production of the carboxymethyl sulfoethyl cellulose CMSEC claimed in claim 1 by etherification of carboxymethyl cellulose CMC in alkaline solution with at least one compound SA transferring a sulfoalkyl group, characterized in that
   a) CMC is suspended in a secondary or tertiary alcohol,
   b) the suspension is combined with the compound SA at pH 0 to 9 and then
   c) alkalized to pH 13-14 by addition of alkali,
   d) the etherification is carried out at a temperature of 55 to 100 °C to form a CMSEC.

**Revendications**

1. Carboxyméthylsulfoéthylcellulose (CMSEC) présentant
   1. un degré de substitution $DS_{sulfoéthyle}$ de 0,1 à 1 et un degré de substitution $DS_{carboxyméthyle}$ de 0,3 à 1,2, les degrés de substitution vérifiant l'équation suivante :

   $0{,}5 \leqq DS_{sulfoéthyle} + DS_{carboxyméthyle} \leqq 1{,}6$,

   2. une viscosité, déterminée sur une solution à 2 % en poids, allant de 5 mPa.s à 60 000 mPa.s (mesurée pour un gradient de cisaillement de 2,5 $s^{-1}$ et à 20 °C) et
   3. une transmission T, déterminée sur une solution aqueuse à 2 % en poids, supérieure à 95 % (longueur d'onde de la lumière utilisée : $\lambda$ = 550 nm, longueur du trajet optique de la cuve = 10 mm), obtenue

   par éthérification de cellulose en solution alcaline avec au moins un composé SA transférant un groupe sulfoalkyle et au moins un composé CM transférant un groupe carboxyméthyle, selon laquelle
   a) de la cellulose est mise en suspension dans un alcool secondaire ou tertiaire,
   b) la suspension est réunie avec le composé SA et/ou CM, avec maintien d'un pH de 0 à 9,
   c) ensuite, le pH est ajusté à une valeur alcaline de 13 à 14 par l'addition d'un alcali,
   d) l'éthérification est conduite à une température comprise entre 55 et 100 °C et
   e) le cas échéant, le composé CM et/ou SA est ajouté ensuite, à des températures de 15 à 100 °C et à un pH de 10 à 14, avec formation d'une CMSEC.

2. Carboxyméthylsulfoéthylcellulose suivant la revendication 1, obtenue par le fait que le composé SA est seul ajouté dans l'étape réactionnelle b) et le composé CM est seul ajouté dans l'étape réactionnelle e).

3. Carboxyméthylsulfoéthylcellulose suivant la revendication 1, obtenue de façon telle que seul le composé CM est ajouté dans l'étape réactionnelle b) et seul le composé SA est ajouté dans l'étape réactionnelle e).

4. Procédé de production d'une carboxyméthylsulfoéthylcellulose CMSEC suivant la revendication 1 par éthérification de carboxyméthylcellulose CMC en solution alcaline avec au moins un composé SA transférant un groupe sulfoalkyle, caractérisé en ce que
   a) de la CMC est mise en suspension dans un alcool secondaire ou tertiaire,
   b) la suspension est réunie avec le composé SA avec maintien d'un pH de 0 à 9,
   c) ensuite, le pH est ajusté à une valeur alcaline de 13 à 14 par addition d'un alcali,
   d) l'éthérification est conduite à une température comprise entre 55 et 100 °C avec formation d'une CMSEC.